# EUROPÄISCHE PATENTSCHRIFT

(11) **EP 0 716 658 B1**
(45) Veröffentlichungstag und Bekanntmachung des Hinweises auf die Patenterteilung: **04.06.1997**
(21) Anmeldenummer: 94926204.2
(22) Anmeldetag: 17.08.1994
(51) Int. Cl.: C07H 15/12, C07K 9/00, A61K 31/70, A61K 38/14

(54) **GLYCOSYLAMIDE VON 2-AMINOACYLAMINO-2-DESOXY-ZUCKERN**
GLYCOSYL AMIDES OF 2-AMINOACYLAMINO-2-DEOXY SUGARS
GLYCOSYLAMIDES DE 2-AMINOACYLAMINO-2-DESOXY-SACCHAROSES

(30) Priorität: 30.08.1993 DE 4329095; 11.02.1994 DE 4404371
(43) Veröffentlichungstag der Anmeldung: 19.06.1996
(73) Patentinhaber: BAYER AG, 51368 Leverkusen (DE)
(72) Erfinder: LOCKHOFF, Oswald, D-51373 Leverkusen (DE); MIELKE, Burkhard, D-51375 Leverkusen (DE); BRUNNER, Helmut, D-42113 Wuppertal (DE); SCHALLER, Klaus, D-42109 Wuppertal (DE)
(86) Internationale Anmeldenummer: EP9402736
(87) Internationale Veröffentlichungsnummer: WO9506654

(56) Entgegenhaltungen:
- EP-A- 0 091 645
- EP-A- 0 338 308
- ANGEWANDTE CHEMIE. INTERNATIONAL EDITION, Bd.30, Nr.12, 1991, WEINHEIM DE Seiten 1611 - 1620 LOCKHOFF O. 'Glycolipids as Immunomodulators: Syntheses and properties'

## Beschreibung

Die Erfindung betrifft am Stickstoff-atom substituierte (2-Aminoacylamino-2-desoxyglycosyl)-amide, Verfahren zu ihrer Herstellung, sowie ihre Verwendung in Arzneimitteln.

Es ist bekannt, daß Glycosylamide von Aldopyranosen oder von Aminozuckern die körpereigene Immunantwort verstärken können (DE-OS 32 13 650). Außerdem ist bekannt, daß mit Aminosäuren substituierte (2-Amino-2-desoxy-glycosyl)-amide sowohl eine Steigerung der spezifischen als auch der unspezifischen Immunantwort bewirken können (DE-OS 35 21 994). In der EP 338 308 werden Glycosylamide beschrieben, die mit substituierten Monoaminosäuren substitutiert sind.

Die vorliegende Erfindung betrifft jetzt am Stickstoff-atom der Aminosäure substituierte (2-Aminoacylamino-2-desoxy-glycosyl)-amide der allgemeinen Formel (I), 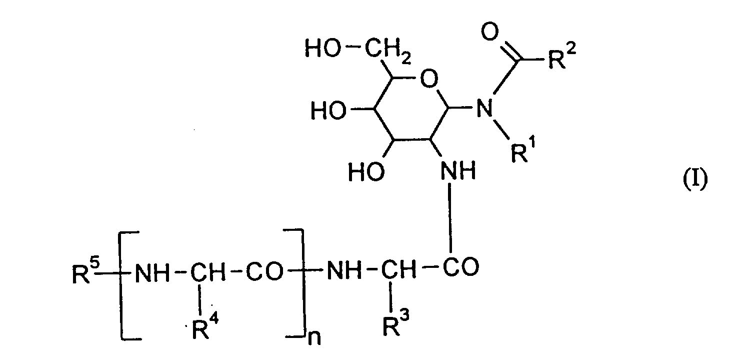 in welcher
- R¹: für geradkettiges oder verzweigtes, gesättigtes oder ungesättigtes Alkyl mit bis zu 25 Kohlenstoffatomen steht,
- R²: für geradkettiges oder verzweigtes, gesättigtes oder ungesättigtes Alkyl mit bis zu 25 Kohlenstoffatomen steht,
- R³: für Wasserstoff, C₁- bis C₇-Alkyl, Hydroxy-methyl, 1-Hydroxy-ethyl, Mercapto-methyl, 2-Methylthio-ethyl, 3-Amino-propyl, 3-Ureido-propyl, 3-Guanidyl-propyl, 4-Amino-butyl, Carboyxy-methyl, Carbamoyl-methyl, 2-Carboxy-ethyl, 2-Carbamoyl-ethyl, Benzyl, 4-Hydroxy-benzyl, 3-Indolylmethyl oder 4-Imidazolyl-methyl steht,
- R⁴: die oben angegebene Bedeutung von R³ hat und mit dieser gleich oder verschiedenen ist,
- R⁵: für Wasserstoff oder eine in der Peptidchemie übliche Schutzgruppe (vergl. A. Hubbuch, Kontakte (Darmstadt) 1979, 14; E. E. Bullesbach, Kontakte (Darmstadt) 1980, 23) steht,
und worin
- n: eine Zahl 1, 2 oder 3 bedeutet,
wobei für den Fall, daß n = 2 oder 3 ist, die einzelnen Bedeutungen von R⁴ verschieden sein können.

Die erfindungsgemäßen Verbindungen haben mehrere asymmetrische Kohlenstoffatome. Sie können daher in verschiedenen stereochemischen Formen existieren. Die Erfindung betrifft sowohl die einzelnen Isomeren als auch deren Mischungen.

Bevorzugt sind Verbindungen der allgemeinen Formel (I), in welcher
- R¹: für einen geradkettigen, gesättigten oder einfach ungesättigten Alkylrest mit 10 bis 20 Kohlenstoffatomen steht,
- R²: für einen geradkettigen, gesättigten oder einfach ungesättigten Alkylrest mit 10 bis 20 Kohlenstoffatomen steht,
- R³: für Wasserstoff, C₁- bis C₇-Alkyl, Hydroxy-methyl, 1-Hydroxy-ethyl, Mercapto-methyl, 2-Methylthio-ethyl, 3-Amino-propyl, 3-Ureido-propyl, 3-Guanidyl-propyl, 4-Amino-butyl, Carboyxy-methyl, Carbamoyl-methyl, 2-Carboxy-ethyl, 2-Carbamoyl-ethyl, Benzyl, 4-Hydroxy-benzyl, 3-Indolyl-methyl oder 4-Imidazolyl-methyl steht,
- R⁴: die oben angegebene Bedeutung von R³ hat und mit dieser gleich oder verschiedenen ist,
- R⁵: für Wasserstoff, Acetyl, Benzoyl, Trichloracetyl, Trifluoracetyl, Methoxycarbonyl, tert.-Butyloxycarbonyl, Allyloxycarbonyl, Trichlorethoxycarbonyl, Benzyloxycarbonyl oder Fluorenylmethyoxycarbonyl steht,
und worin
- n: eine Zahl 1, 2 oder 3 bedeutet,
wobei für den Fall, daß n = 2 oder 3 ist, die einzelnen Bedeutungen von R⁴ verschieden sein können.

Außerdem wurden zwei Verfahren der Herstellung der erfindungsgemäßen Verbindungen der allgemeinen Formel (I) gefunden. Die beiden Verfahren unterscheiden sich, in welcher Reihenfolge und mit welchen Bausteinen die peptidischen Bindungen geknüpft werden.

In dem ersten Verfahren (A) werden N-(2-Aminoacylamino-2-desoxy-hexopyranosyl)-N-alkyl-carbonsäureamide der Formel (II) 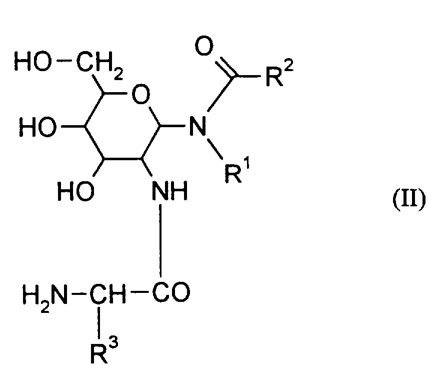 in welcher
R¹, R² und R³ die oben angegebene Bedeutung haben,
mit Aminosäure-, Di- oder Tripeptid-derivaten der allgemeinen Formel (III) 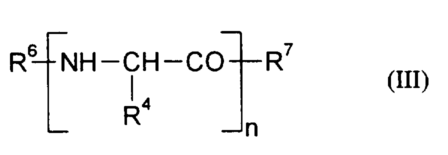 in welcher
- R⁴: die oben angegebene Bedeutung hat,
- R⁶: eine in der Peptidchemie übliche Schutzgruppe für das Stickstoff-atom von Aminosäuren darstellt, die selektiv unter Erhalt der Peptidbindung wieder abgespalten werden kann, und
- R⁷: eine Hydroxygruppe oder eine in der Peptidchemie übliche Fluchtgruppe für die Aktivierung von Aminosäuren darstellt,
- n: eine Zahl 1, 2 oder 3 bedeutet,
wobei für den Fall, daß n = 2 oder 3 ist, die einzelnen Bedeutungen von R⁴ verschieden sein können, miteinander derart zur Reaktion bringt, daß eine amidische Bindung geknüpft wird und Verbindungen der allgemeinen Formel (I) erhalten werden.

In einem zweiten Reaktionsschritt wird die N-terminale Schutzgruppe R⁵ in den Verbindungen der Formel (I) abgespalten, wobei die Verbindungen der allgemeinen Formel (I) mit einer freien Aminogruppe erhalten werden.

Die Ausgangsverbindungen der allgemeinen Formel (II) sind bekannt und können nach den in DE3521994 (LeA 23620) beschriebenen Verfahren hergestellt werden.

Die Derivate der Di- oder Tripeptide der allgemeinen Formel (III) sind ebenfalls prinzipiell bekannt.

Geeignete Schutzgruppen R⁶ für die Aminofunktion in Verbindungen der Formel (III) sind z.B. Acylgruppen wie Trifluoracetyl oder Trichloracetyl, o-Nitrophenylsulfenyl, 2,4-Dinitrophenylsulfenyl oder gegebenenfalls substituiertes Niederalkoxycarbonyl wie z.B. Methoxycarbonyl, tert.-Butyloxycarbonyl, Benzyloxycarbonyl, p-Methoxybenzyloxycarbonyl, Fluorenylmethoxycarbonyl oder 2,2,2-Trichlorethoxycarbonyl.

Bevorzugte Aminoschutzgruppen R⁶ sind die tert.-Butyloxycarbonyl-gruppe oder die Benzyloxycarbonyl-gruppe.

Die Verknüpfung der 2-Aminoacylamino-2-desoxy-glycosylamide der allgemeinen Formel (II) mit den N-substituierten Aminosäuren, Di- oder Tripeptiden der allgemeinen Formel (III) kann nach gängigen Methoden der Peptidchemie erfolgen (E. Wünsch et al.: Synthese von Peptiden, in: Methoden der Organischen Chemie (Houben-Weyl) (E. Müller, Hrsg.) Band XV/I und XV/II, 4. Aufl., Thieme Verlag Stuttgart (1974)).

Gängige Verfahren sind z.B. die Kondensation der Aminofunktion in der Verbindung der allgemeinen Formel (II) mit einem N-geschützten Aminosäure- oder Peptidderivat der allgemeinen Formel (III) in Gegenwart von wasserentziehenden Mitteln, z.B. Carbodiimiden wie Dicyclohexylcarbodiimid oder Diisopropylcarbodiimid.

Die Kondensation der Verbindungen der Formel (II) mit den Verbindungen der Formel (III) kann auch durchgeführt werden, wenn die Carboxygruppe aktiviert ist. Eine aktivierte Carboxygruppe kann z.B. ein Carbonsäureanhydrid sein, bevorzugt ein gemischtes Anhydrid mit Alkylcarbonaten, Essigsäure oder einer anderen Carbonsäure, oder ein Amid der Säure, wie ein Imidazolid, oder ein aktivierter Ester wie z.B. Cyanomethylester, Pentachlorphenylester oder N-Hydroxyphthalimidester. Aktivierte Ester können auch aus den Aminosäurederivaten der Formel (III), in denen R⁷ für OH steht, und N-Hydroxysuccinimid oder 1-Hydroxybenzotriazol in Gegenwart eines wasserentziehenden Mittels wie Carbodiimid erhalten werden.

In dem zweiten Verfahrensschritt zur Darstellung der Verbindungen der allgemeinen Formel (I) wird die Schutzgruppe R⁵ abgespalten.

Die bevorzugt verwendeten Schutzgruppen R⁵ in den Verbindungen der allgemeinen Formel (I), die N-Carbobenzoxy-gruppe und die N-tert.-Butyloxycarbonyl-gruppe, lassen sich unter Erhalt der in den Verbindungen vorliegenden amidischen Gruppen abspalten. Derartige Verfahren sind prinzipiell bekannt.

Die Carbobenzoxy-gruppe in den Verbindungen der Formel (I) läßt sich selektiv durch Hydrogenolyse in Gegenwart von Übergangsmetallen, wie z.B. Palladium auf Kohle, in einem geeigneten Lösungsmittel wie z.B. Methanol, Ethanol, Eisessig oder Tetrahydrofuran, entweder in reiner Form oder in Kombination der Lösungsmittel untereinander, oder auch Wasser abspalten, wobei sowohl bei Normaldruck als auch bei erhöhtem Druck gearbeitet werden kann.

Die tert.-Butyloxycarbonyl-gruppe in den Verbindungen der Formel (I) läßt sich mittels acidolytischer Verfahren abspalten. Geeignete Bedingungen sind z.B. die Verwendung von Chlorwasserstoff oder Trifluoressigsäure, entweder in reiner Form oder verdünnt in geeigneten Lösungsmitteln wie z.B. Eisessig, Dichlormethan, Diethylether, Dioxan oder Essigsäureethylester.

Die auf diese Weise erhaltenen Verbindungen der allgemeinen Formel (I) werden nach an sich bekannten Verfahren in Form von kristallinen oder amorphen Feststoffen isoliert und werden, falls notwendig, durch Umkristallisation, Chromatographie, Extraktion usw. gereinigt.

In dem zweiten Verfahren (B) werden die erfindungsgemäßen Verbindungen der Formel (I) dadurch erhalten, daß man N-(2-Amino-2-desoxy-hexopyranosyl)-N-alkylcarbonsäureamide mit Di-, Tri- oder Tetrapeptid-derivaten verknüpft.

Das Verfahren (B) ist dadurch gekennzeichnet, daß man Verbindungen der allgemeinen Formel (IV) 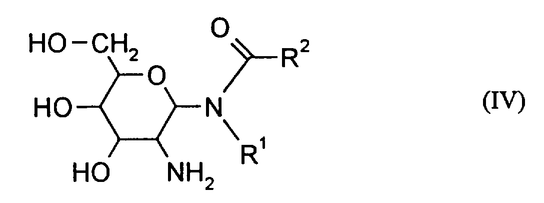 in welcher
R¹ und R² die oben angegebene Bedeutung haben
mit Derivaten von Di-, Tri- oder Tetrapeptiden der allgemeinen Formel (III) 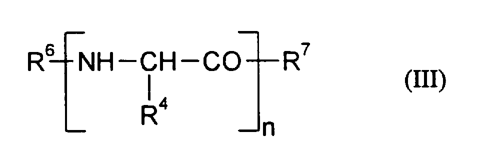 in welcher
- R⁴: die oben angegebene Bedeutung hat und
- R⁶: eine Schutzgruppe bedeutet
und worin
- n: eine Zahl 2, 3 oder 4 bedeutet
miteinander unter den oben genannten Bedingungen unter Ausbildung einer peptidischen Bindung zu Reaktion bringt, wobei die an der N-terminalen Aminogruppe substituierten Verbindungen der Formel (V) 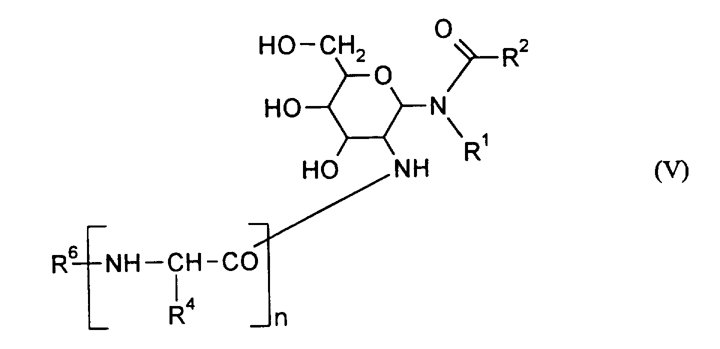 worin R¹, R², R⁴ und R⁶ die oben angegebenen Bedeutungen haben, und
- n: eine Zahl 2, 3 oder 4 bedeutet,
erhalten werden.

In den substituierten Verbindungen der Formel (V) sind anschließend die Amino-schutzgruppen R⁶ abzuspalten, wobei Verbindungen mit unsubstituierter Aminogruppe der allgemeinen Formel (I) erhalten werden.

Die Herstellung der Ausgangsverbindungen der Formel (IV) ist in DE3521994 (LeA 23620) beschrieben. Für die Bedingungen der Knüpfung der peptidischen Bindungen können die oben angegebenen allgemeinen Vefahren der Peptidsynthese angewendet werden.

Zum Gegenstand der Erfindung gehören auch Salze der Verbindungen der Formel (I). Dabei handelt es sich in erster Linie um üblicherweise pharmazeutisch verwendbare, nicht-toxische Salze, z.B. Ammoniumsalze der Chloride, Acetate, Lactate.

Es wurde gefunden, daß die im folgenden näher bezeichneten Verbindungen der allgemeinen Formel (I) eine Stimulierung und damit eine Verbesserung körpereigener Abwehrvorgänge bewirken. Die Verbindungen können daher als immunologisch aktive Medikamente verwendet werden. Die immunstimulierende Wirkung wurde sowohl in vivo im Tierexperiment als auch in vitro an Zellen des Abwehrsystems nachgewiesen. Diese Tatsache wird durch folgende Versuchsergebnisse belegt.

Weibliche Mäuse (CFW₁) mit einem Gewicht von ca. 18 g wurden nach Zufallskriterien auf Gruppen verteilt. Die Tiere wurden dann intraperitoneal, subcutan oder intravenös mit einer Dosis von 10 mg/kg Körpergewicht der erfindungsgemäßen Verbindungen der Formel (I) behandelt, oder erhielten physiologische Kochsalz-Lösung. Vierundzwanzig Stunden später wurden die Tiere mit der 10-fachen letalen Dosis (LD₅₀) von Escherichia coli C14 intraperitoneal infiziert. Die folgende Tabelle zeigt, daß die Überlebensraten sieben Tage nach der Infektion bei Mäusen, die mit den erfindungsgemäßen Verbindungen der Formel (I) behandelt worden waren, signifikant über der von Mäusen lag, die physiologische Kochsalz-Lösung erhalten hatten.

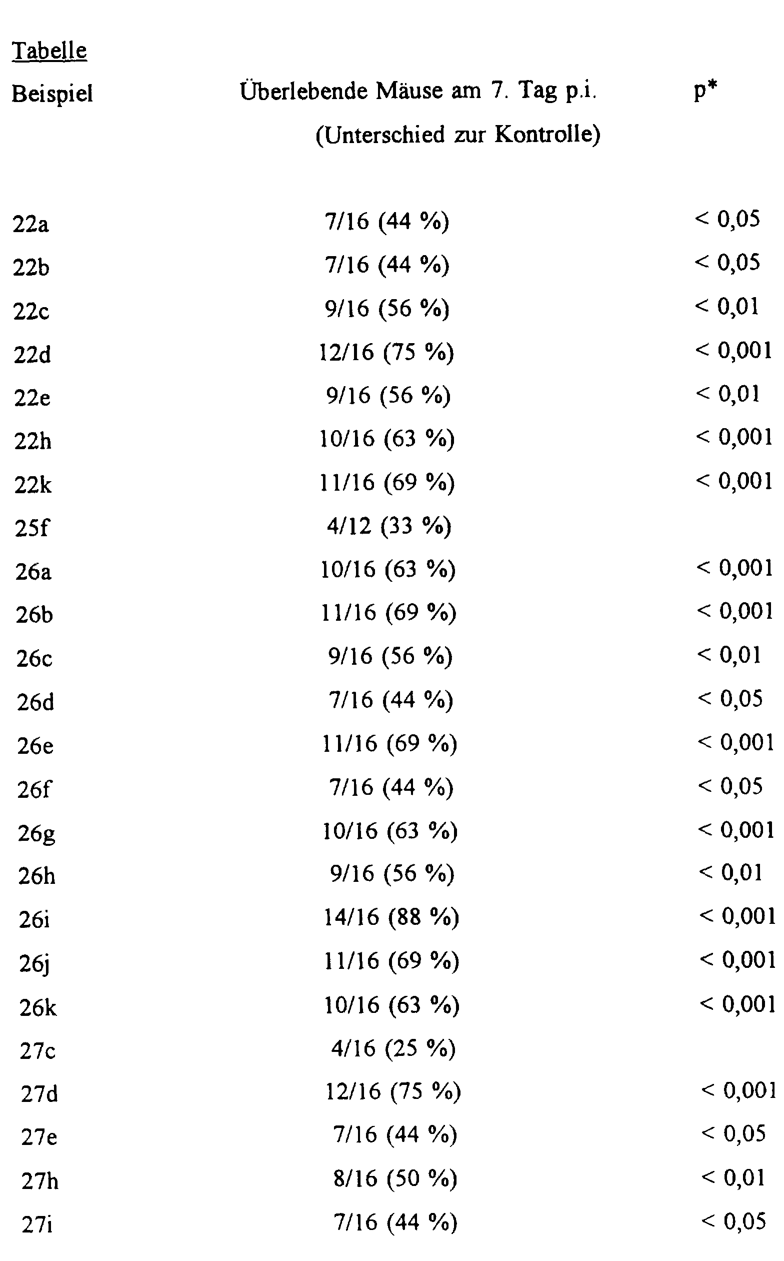

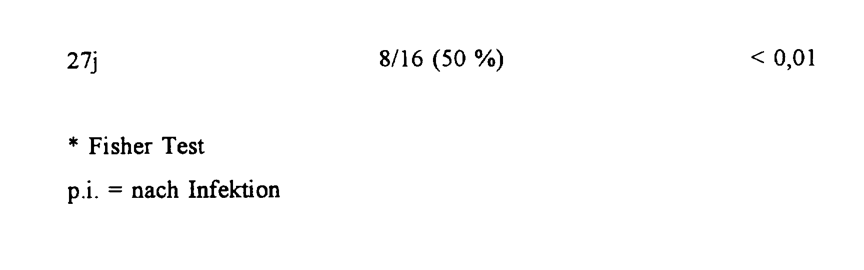

### Protektives Screening im neutropenischen Candida - Infektionsmodell

Ziel dieses Versuchsmodells ist das Auffinden von Substanzen, die die körpereigene Abwehr bei neutropenischen Mäusen stimulieren.

### Methodik

Zum Zeitpunkt - 96 h werden die Mäuse mit 0,2 ml Endoxan in der Dosis 200 mg/kg pro Tier intra peritoneal behandelt. Zu den Zeitpunkten - 72, - 48 und - 24 h werden die Mäuse mit 0,2 ml Lösung der Screening-Substanz intra peritoneal behandelt. Alternativ werden Behandlungen mit Screening-Substanzen auch mit 0,2 ml Lösung s.c. und i.v., als auch mit 0,5 ml Lösung per os durchgeführt. Pro Präparat werden 2 Gruppen mit je 10 Tieren eingesetzt. Routinemäßig wird eine Gruppe mit 10 mg/kg, die andere mit 30 mg/kg Körpergewicht behandelt. Für vertiefte Prüfungen werden auch wesentlich niedrigere Dosierungen eingesetzt.

Zum Zeitpunkt 0 h werden die Mäuse mit 0,2 ml einer letalen Keimaufschwemmung i.v. in die Schwanzvene infiziert.

### Beobachtung und Beurteilung

Die Mäuse werden bis 4h nach der Behandlung beobachtet, um mögliche Präparatunverträglichkeiten erfassen zu können.

Ab 1 Tag bis 14 Tage nach der Infektion, werden die Mäuse 1 x täglich vormittags beurteilt. Der Gesundheitszustand wird in 5 Abstufungen erfaßt.
( - gut - leichtkrank - krank - schwerkrank - tot -)
Die schwerkranken Mäuse werden nach der Beurteilung abgetötet, sie sollen nicht leiden.

### Ergebnis

Als Ergebnis wird die Überlegungsquote und/oder eine Verzögerung des Krankheitsbildes dokumentiert. Beides im Vergleich zu den mit Endoxan behandelten Kontroll-Mäusen.

### Techniken

Applikationen i.p., s.c. und i.v. führen wir mit einer 1 ml Einwegspritze und einer Injektionsspritze Nr. 18 durch. Die per os Applikation wird mit einer 5 ml Einwegspritze und der Injektionsspritze Nr. 12 mit Olive durchgeführt. Zur i.p. und per os Applikation werden die Mäuse in der Hand fixiert. Bei der s.c-Applikation werden die Mäuse auf dem Käfigdeckel fixiert. Die Mäuse werden bei der i.v.-Applikation und i.v.-Infektion in einem Mäusezwangskäfig fixiert. Außerdem werden die Mäuse vor der i.v.-Behandlung und -Infektion ca. 10 Minuten unter Rotlicht gestellt, um die Schwanzvenen zu weiten.

### Parameter

- Maus:: B₆D₂F₁ 20 g, weiblich
- Keim:: Candida albicans, 5 x 10⁵ Keime pro Maus
- Substanz:: Endoxan ist wasserlöslich.
Die Screening-Substanzen werden, wenn möglich, ebenfalls in sterilem Wasser gelöst. Ist dies nicht möglich, wird versucht, sie wie folgt zu lösen: anlösen in reinem DMSO (DMSO-Endkonzentration in der Applikationslösung = 2 %). Danach erfolgte die Zugabe von Cremophor (Cremophor-Endkonzentration in der Applikationslösung = 8 %).
Mit sterilem Wasser wird auf das Endvolumen aufgefüllt.
- Tierhaltung:: Die Tiere werden in Typ II Makrolonkäfigen gehalten. Alle Tiere erhalten Futter und Wasser ad libitum.

Die Verbindung aus dem Beispiel 22 h zeigt eine gute protektive Wirkung im Candida-Infektionsmodell.

### Herstellungsbeispiele

### Beispiel 1

Allgemeine Vorschrift zur Umsetzung der 2-Amino-2-desoxy-verbindungen der Formel (IV) bzw. der 2-Aminoacylamino-2-desoxy-verbindungen der Formel (II) mit N-geschützten Aminosäuren, Di- oder Tripeptiden der allgemeinen Formel (III) zu den N-geschützten, mit Aminosäuren substituierten 2-Aminoacylamino-2-desoxyverbindungen der Formel (I):

Die Mischung der N-geschützten α-Aminosäure, bzw. des Di- oder Oligopeptids (7,7 mmol), N-Hydroxysuccinimid (1,77 g, 15,4 mmol) und N,N-Dimethylformamid (70 ml) wird mit N,N-Dicyclohexylcarbodiimid (1,88 g, 8,4 mmol) versetzt und 30 min bei 20° gerührt. Es wird mit Ethyl-diisopropylamin (8,0 mmol) und mit der 6-Amino-6-desoxy-verbindung gemäß Beispiel II (7,0 mmol) versetzt und 16 h gerührt. Die Mischung wird mit Wasser (3 ml) versetzt, 30 min nachgerührt und bei vermindertem Druck zum Sirup eingeengt. Der Rückstand wird mit Diethylether (100 ml) verrührt, der ausgefallene Harnstoff wird abgesaugt, das Filtrat wird im Hochvakuum eingeengt. Der Rückstand wird in Diethylether (150 ml) aufgenommen, dreimal mit Wasser (je 70 ml) gewaschen, über Magnesiumsulfat getrocknet und bei vermindertem Druck zum Sirup eingeengt. Der Rückstand wird über Kieselgel filtriert (Laufmittel Dichlormethan/Methanol/konz. Ammoniak-Wasser 25:1:0,05).
- 11a: N-[2-(N-Carbobenzoxy-glycyl-glycyl)-amino-2-desoxy-β-D-glucopyranosyl]-N-dodecyl-dodecansäureamid.
aus N-(2-Glycyl-amino-2-desoxy-β-D-glucopyranosyl)-N-dodecyl-dodecansäureamid und N-Carbobenzoxy-glycin.
- 11b: N-[2-(N-Carbobenzoxy-L-alanyl-glycyl)-amino-2-desoxy-β-D-glucopyranosyl]-N-dodecyl-dodecansäureamid.
aus N-(2-Glycyl-amino-2-desoxy-β-D-glucopyranosyl)-N-dodecyl-dodecartsäureamid und N-Carbobenzoxy-L-alanin.
- 11c: N-[2-(N-Carbobenzoxy-L-phenylalanyl-glycyl)-amino-2-desoxy-β-D-glucopyranosyl]-N-dodecyl-dodecansäureamid.
aus N-(2-Glycyl-amino-2-desoxy-β-D-glucopyranosyl)-N-dodecyl-dodecansäureamid und N-Carbobenzoxy-L-phenylalanin. Ausbeute 53%. R_{f}-Wert 0,29 (Dichlormethan/Methanol/konz. Ammoniak 15:1:0,1).
- 11d: N-[2-(N-Carbobenzoxy-glycyl-L-alanyl)-amino-2-desoxy-β-D-glucopyranosyl]-N-dodecyl-dodecansäureamid.
aus N-(2-L-Alanyl-amino-2-desoxy-β-D-glucopyranosyl)-N-dodecyl-dodecansäureamid und N-Carbobenzoxy-glycin.
- 11e: N-[2-(N-Carbobenzoxy-glycyl-D-alanyl)-amino-2-desoxy-β-D-glucopyranosyl]-N-dodecyl-dodecansäureamid.
aus N-(2-D-Alanyl-amino-2-desoxy-β-D-glucopyranosyl)-N-dodecyl-dodecansäureamid und N-Carbobenzoxy-glycin.
- 11f: N-[2-(N-Carbobenzoxy-4-O-benzyl-L-aspartyl-D-alanyl)-amino-2-desoxy-β-D-glucopyranosyl]-N-dodecyl-dodecansäureamid.
aus N-(2-D-Alanyl-amino-2-desoxy-β-D-glucopyranosyl)-N-dodecyl-dodecansäureamid und N-Carbobenzoxy-4-O-benzyl-L-asparaginsäure. Ausbeute 63%. R_{f}-Wert 0,57 (Dichlormethan/Methanol/konz. Ammoniak 15:1:0,1).
- 12a: N-[2-(N-Carbobenzoxy-glycyl-glycyl)-amino-2-desoxy-β-D-glucopyranosyl]-N-tetradecyl -dodecansäureamid.
aus N-(2-Glycyl-amino-2-desoxy-β-D-glucopyranosyl)-N-tetradecyl-dodecansäureamid und N-Carbobenzoxy-glycin. Ausbeute 47 %. [α]_{D} = +18,7° (c = 0,95, Dichlormethan). Schmp. 108-109°.
- 12b: N-[2-(N-Carbobenzoxy-sarcosyl-glycyl)-amino-2-desoxy-β-D-glucopyranosyl]-N-tetradecyl-dodecansäureamid.
aus N-(2-Glycyl-amino-2-desoxy-β-D-glucopyranosyl)-N-tetradecyl-dodecansäureamid und N-Carbobenzoxy-sarcosin. Ausbeute 64 %. [α]_{D} = +18,4° (c = 1,02, Dichlormethan). Schmp. 76-77°.
- 12c: N-[2-(N-Carbobenzoxy-L-alanyl-glycyl)-amino-2-desoxy-β-D-glucopyranosyl]-N-tetradecyl-dodecansäureamid.
aus N-(2-Glycyl-amino-2-desoxy-β-D-glucopyranosyl)-N-tetradecyl -dodecansäureamid und N-Carbobenzoxy-L-alanin. Ausbeute 94 %. [α]_{D} = +16,3° (c = 1,12, Dichlormethan). Schmp. 103-105°.
- 12d: N-[2-(N-Carbobenzoxy-L-valyl-glycyl)-amino-2-desoxy-β-D-glucopyranosyl]-N-tetradecyl-dodecansäureamid.
aus N-(2-Glycyl-amino-2-desoxy-β-D-glucopyranosyl)-N-tetradecyl-dodecansäureamid und N-Carbobenzoxy-L-valin. Ausbeute 76 %. [α]_{D} = +17,8° (c = 1,09, Dichlormethan). Schmp. 85-86°.
- 12e: N-[2-(N-Carbobenzoxy-L-seryl-glycyl)-amino-2-desoxy-β-D-glucopyranosyl]-N-tetradecyl -dodecansäureamid.
aus N-(2-Glycyl-amino-2-desoxy-β-D-glucopyranosyl)-N-tetradecyl-dodecansäureamid und N-Carbobenzoxy-L-serin. Ausbeute 76 %. [α]_{D} = +20,3° (c = 0,89, Dichlormethan). Schmp. 87-89°.
- 12f: N-[2-(N-Carbobenzoxy-O-benzyl-L-glutamyl-glycyl)-amino-2-desoxy-β-D-glucopyranosyl] -N-tetradecyl-dodecansäureamid.
ausN-(2-Glycyl-amino-2-desoxy-β-D-glucopyranosyl)-N-tetradecyl-dodecansäureamid und N-Carbobenzoxy-O-benzyl-L-glutaminsäure.
- 12g: N-[2-(N-Carbobenzoxy-L-glutaminyl-glycyl)-amino-2-desoxy-β-D-glucopyranosyl]-N-tetradecyl-dodecansäureamid.
ausN-(2-Glycyl-amino-2-desoxy-β-D-glucopyranosyl)-N-tetradecyl-dodecansäureamid und N-Carbobenzoxy-L-glutamin.
- 12h: N-[2-(N-Carbobenzoxy-glycyl-glycyl-glycyl)-amino-2-desoxy-β-D-glucopyranosyl]-N-tetradecyl-dodecansäureamid.
ausN-(2-Glycyl-amino-2-desoxy-β-D-glucopyranosyl)-N-tetradecyl-dodecansäureamid und N-Carbobenzoxy-glycyl-glycin. Ausbeute 52 %. [α]_{D} = +14,7° (c = 1,01, Tetrahydrofuran). Schmp. 99-100°.
- 12i: N-[2-(N-Carbobenzoxy-glycyl-glycyl-glycyl-glycyl)-amino-2-desoxy-β-D-glucopyranosyl]-N-tetradecyl-dodecansäureamid.
ausN-(2-Glycyl-amino-2-desoxy-β-D-glucopyranosyl)-N-tetradecyl-dodecansäureamid und N-Carbobenzoxy-glycyl-glycyl-glycin.
- 12j: N-[2-(N-Carbobenzoxy-L-alanyl-glycyl-glycyl)-amino-2-desoxy-β-D-glucopyranosyl]-N-tetradecyl-dodecansäureamid.
ausN-(2-Glycyl-amino-2-desoxy-β-D-glucopyranosyl)-N-tetradecyl-dodecansäureamid und N-Carbobenzoxy-L-alanyl-glycin.
- 12k: N-[2-(N-Carbobenzoxy-L-alanyl-L-alanyl-glycyl)-amino-2-desoxy-β-D-glucopyranosyl]-N-tetradecyl-dodecansäureamid.
ausN-(2-Glycyl-amino-2-desoxy-β-D-glucopyranosyl)-N-tetradecyl-dodecansäureamid und N-Carbobenzoxy-L-alanyl-L-alanin. Ausbeute 70 %. [α]_{D} = +10,3° (c = 0,92, Tetrahydrofuran). Schmp. 99-100°.
- 13a: N-[2-(N-Carbobenzoxy-glycyl-glycyl)-amino-2-desoxy-β-D-glucopyranosyl]-N-octadecyl-dodecansäureamid.
aus N-(2-Glycyl-amino-2-desoxy-β-D-glucopyranosyl)-N-octadecyldodecansäureamid und N-Carbobenzoxy-glycin.
- 13b: N-[2-(N-Carbobenzoxy-L-valyl-glycyl)-amino-2-desoxy-β-D-glucopyranosyl]-N-octadecyl-dodecansäureamid.
aus N-(2-Glycyl-amino-2-desoxy-β-D-glucopyranosyl)-N-octadecyl-dodecansäureamid und N-Carbobenzoxy-L-valin.
- 13c: N-[2-(N-Carbobenzoxy-L-leucyl-glycyl)-amino-2-desoxy-β-D-glucopyranosyl]-N-octadecyl-dodecansäureamid.
aus N((2-Glycyl-amino-2-desoxy-β-D-glucopyranosyl)-N-octadecyl-dodecansäureamid und N-Carbobenzoxy-L-leucin.
- 13d: N-[2-(N-Carbobenzoxy-O-benzyl-L-glutamyl-glycyl)-amino-2-desoxy-β-D-glucopyranosyl]-N-octadecyl-dodecansäureamid.
aus N-(2-Glycyl-amino-2-desoxy-β-D-glucopyranosyl)-N-octadecyl-dodecansäureamid und N-Carbobenzoxy-O-benzyl-L-glutaminsäure.
- 13e: N-[2-(N-Carbobenzoxy-L-glutaminyl-glycyl)-amino-2-desoxy-β-D-glucopyranosyl]-N-octadecyl-dodecansäureamid.
aus N-(2-Glycyl-amino-2-desoxy-β-D-glucopyranosyl)-N-octadecyl-dodecansäureamid und N-Carbobenzoxy-L-glutamin.
- 13f: N-[2-(Tri-N-Carbobenzoxy-L-arginyl-glycyl)-amino-2-desoxy-β-D-glucopyranosyl]-N-octadecyl-dodecansäureamid.
aus N-(2-Glycyl-amino-2-desoxy-β-D-glucopyranosyl)-N-octadecyl-dodecansäureamid und Tri-N-carbobenzoxy-L-arginin.
- 14a: N-[2-(N-Carbobenzoxy-glycyl-glycyl)-amino-2-desoxy-β-D-glucopyranosyl]-N-tetradecyl-octadecansäureamid.
aus N-(2-Glycyl-amino-2-desoxy-β-D-glucopyranosyl)-N-tetradecyl-octadecansäureamid und N-Carbobenzoxy-glycin.
- 14b: N-[2-(N-Carbobenzoxy-glycyl-L-alanyl)-amino-2-desoxy-β-D-glucopyranosyl]-N-tetradecyl-octadecansäureamid.
aus N-(2-L-Alanyl-amino-2-desoxy-β-D-glucopyranosyl)-N-tetradecyl-octadecansäureamid und N-Carbobenzoxy-glycin.
- 14c: N-[2-(N-Carbobenzoxy-L-leucyl-L-alanyl)-amino-2-desoxy-β-D-glucopyranosyl]-N-tetradecyl-octadecansäureamid.
aus N-(2-L-Alanyl-amino-2-desoxy-β-D-glucopyranosyl)-N-tetradecyl-octadecansäureamid und N-Carbobenzoxy-L-leucin.
- 14d: N-[2-(N-Carbobenzoxy-L-alanyl-L-leucyl)-amino-2-desoxy-β-D-glucopyranosyl]-N-tetradecyl-octadecansäureamid.
aus N-(2-L-Leucyl-amino-2-desoxy-β-D-glucopyranosyl)-N-tetradecyl-octadecansäureamid und N-Carbobenzoxy-L-alanin.
- 14e: N-[2-(N-Carbobenzoxy-L-leucyl-L-leucyl)-amino-2-desoxy-β-D-glucopyranosyl]-N-tetradecyl-octadecansäureamid.
aus N-(2-L-Leucyl-amino-2-desoxy-β-D-glucopyranosyl)-N-tetradecyl-octadecansäureamid und N-Carbobenzoxy-L-leucin. Ausbeute 61 %. R_{f}-Wert 0,50 (Dichlormethan/Methanol/konz. Ammoniak 15:1:0,1).
- 15a: N-[2-(N-Carbobenzoxy-glycyl-glycyl)-amino-2-desoxy-β-D-glucopyranosyl]-N-octadecyl -octadecansäureamid.
aus N-(2-Glycyl-amino-2-desoxy-β-D-glucopyranosyl)-N-octadecyl-octadecansäureamid und N-Carbobenzoxy-glycin.
- 15b: N-[2-(N-Carbobenzoxy-L-alanyl-L-alanyl)-amino-2-desoxy-β-D-glucopyranosyl]-N-octadecyl-octadecansäureamid.
aus N-(2-Amino-2-desoxy-β-D-glucopyranosyl)-N-octadecyl-octadecansäureamid und N-Carbobenzoxy-L-alanyl-L-alanin.
- 15c: N-[2-(N-Carbobenzoxy-D-alanyl-L-alanyl)-amino-2-desoxy-β-D-glucopyranosyl]-N-octadecyl-octadecansäureamid.
aus N-(2-Amino-2-desoxy-β-D-glucopyranosyl)-N-octadecyl-octadecansäureamid und N-Carbobenzoxy-D-alanyl-L-alanin.
- 15d: N-[2-(N-Carbobenzoxy-D-alanyl-D-alanyl)-amino-2-desoxy-β-D-glucopyranosyl]-N-octadecyl-octadecansäureamid.
aus N-(2-Amino-2-desoxy-β-D-glucopyranosyl)-N-octadecyl-octadecansäureamid und N-Carbobenzoxy-D-alanyl-D-alanin.
- 15e: N-[2-(N-Carbobenzoxy-D-alanyl-L-alanyl)-amino-2-desoxy-β-D-glucopyranosyl]-N-octadecyl-octadecansäureamid.
aus N-(2-Amino-2-desoxy-β-D-glucopyranosyl)-N-octadecyl-octadecansäureamid und N-Carbobenzoxy-D-alanyl-L-alanin.
- 15f: N-[2-(Di-N-tert.-butyloxycarbonyl-L-lysyl-L-alanyl)-amino-2-desoxy-β-D-glucopyranosyl]-N-octadecyl-octadecansäureamid.
aus N-(2-Amino-2-desoxy-β-D-glucopyranosyl)-N-octadecyl-octadecansäureamid und Di-N-tert.-butyloxycarbonyl-L-lysyl-L-alanin.
- 16a: N-[2-(N-tert.-Butyloxycarbonyl-glycyl-glycyl)-amino-2-desoxy-β-D-glucopyranosyl]-N-tetradecyl-ölsäureamid.
aus N-(2-Glycyl-amino-2-desoxy-β-D-glucopyranosyl)-N-tetradecyl-ölsäureamid und N-tert.-Butyloxycarbonyl-glycin. Ausbeute 68 %. [α]_{D} = +14,5° (c = 0,92, Tetrahydrofuran).
- 16b: N-[2-(N-tert.-Butyloxycarbonyl-L-alanyl-glycyl)-amino-2-desoxy-β-D-glucopyranosyl]-N-tetradecyl-ölsäureamid.
aus N-(2-Glycyl-amino-2-desoxy-β-D-glucopyranosyl)-N-teuadecyl-ölsäureamid und N-tert.-Butyloxycarbonyl-L-alanin. Ausbeute 79 %. [α]_{D} = +12,1° (c = 0,87, Tetrahydrofuran).
- 16c: N-[2-(N-tert.-Butyl oxycarbonyl-glycyl-glycyl-glycyl)-amino-2-desoxy-β-D-glucopyranosyl]-N-tetradecyl-ölsäureamid.
aus N-(2-Glycyl-amino-2-desoxy-β-D-glucopyranosyl)-N-tetradecyl-ölsäureamid und N-tert.-Butyloxycarbonyl-glycyl-glycin. Ausbeute 74 %. [α]_{D} = +14,2° (c = 1,00, Tetrahydrofuran).
- 16d: N-[2-(N-tert.-Butyloxycarbonyl-L-alanyl-L-alanyl-glycyl)-amino-2-desoxy-β-D-glucopyranosyl]-N-tetradecyl-ölsäureamid.
aus N-(2-Glycyl-amino-2-desoxy-β-D-glucopyranosyl)-N-tetradecyl-ölsäureamid und N-tert.-Butyloxycarbonyl-L-alanyl-L-alanin. Ausbeute 71 %. [α]_{D} = +7,4° (c = 0,82, Tetrahydrofuran).
- 16e: N-[2-(N-tert.-Butyloxycarbonyl-glycyl-L-alanyl)-amino-2-desoxy-β-D-glucopyranosyl]-N-tetradecyl-ölsäureamid.
aus N-(2-L-Alanyl-amino-2-desoxy-β-D-glucopyrartosyl)-N-tekadecyl-ölsäureamid und N-tert.-Butyloxycarbonyl-glycin. Ausbeute 61 %. [α]_{D} = +1,2° (c = 0,86, Tetrahydrofuran).
- 16f: N-[2-(N-tert.-Butyloxycarbonyl-L-alanyl-L-alanyl)-amino-2-desoxy-β-D-glucopyranosyl]-N-tetradecyl-ölsäureamid.
aus N-(2-L-Alanyl-amino-2-desoxy-β-D-glucopyranosyl)-N-tekadecyl-ölsäureamid und N-tert.-Butyloxycarbonyl-L-alanin. Ausbeute 59 %. [α]_{D} = +0,2° (c = 0,86, Tetrahydrofuran).
- 16g: N-[2-(N-tert.-Butyloxycarbonyl-L-leucyl-L-alanyl)-amino-2-desoxy-β-D-glucopyranosyl]-N-tetradecyl-ölsäureamid.
aus N-(2-L-Alanyl-amino-2-desoxy-β-D-glucopyranosyl)-N-tetradecyl-ölsäureamid und N-tert.-Butyloxycarbonyl-L-leucin. Ausbeute 56 %. [α]_{D} = -2,7° (c = 0,86, Tetrahydrofuran).
- 16h: N-[2-(N-tert.-Butyloxycarbonyl-glycyl-glycyl-L-alanyl)-amino-2-desoxy-β-D-glucopyranosyl]-N-tetradecyl-ölsäureamid.
aus N-(2-L-Alanyl-amino-2-desoxy-β-D-glucopyranosyl)-N-tetradecyl-ölsäureamid und N-tert.-Butyloxycarbonyl-glycyl-glycin. Ausbeute 56 %. [α]_{D} = +8,5° (c = 0,82, Tetrahydrofuran).
- 16i: N-[2-(N-tert.-Butyloxycarbonyl-glycyl-L-leucyl)-amino-2-desoxy-β-D-glucopyranosyl]-N-tetradecyl-ölsäureamid.
aus N-(2-L-Leucyl-amino-2-desoxy-β-D-glucopyranosyl)-N-tekadecyl-ölsäureamid und N-tert.-Butyloxycarbonyl-glycin. Ausbeute 79 %. [α]_{D} = -1,4° (c = 0,87, Tetrahydrofuran).
- 16j: N-[2-(N-tert.-Butyloxycarbonyl-L-alanyl-L-leucyl)-amino-2-desoxy-β-D-glucopyranosyl]-N-tetradecyl-ölsäureamid.
aus N-(2-L-Leucyl-amino-2-desoxy-β-D-glucopyranosyl)-N-tetradecyl-ölsäureamid und N-tert.-Butyloxycarbonyl-L-alanin. Ausbeute 90 %. [α]_{D} = -8,1° (c = 0,98, Tetrahydrofuran).
- 16k: N-[2-(N-tert.-Butyloxycarbonyl-glycyl-glycyl-L-leucyl)-amino-2-desoxy-β-D-glucopyranosyl]-N-tetradecyl-ölsäureamid.
aus N-(2-L-Leucyl-amino-2-desoxy-β-D-glucopyranosyl)-N-tetradecyl-ölsäureamid und N-tert.-Butyloxycarbonyl-glycyl-glycin. Ausbeute 92 %. [α]_{D} = +4,2° (c = 0,83, Tetrahydrofuran).
- 17a: N-[2-(N-tert.-Butyloxycarbonyl-glycyl-glycyl)-amino-2-desoxy-β-D-glucopyranosyl]-N-octadecyl-ölsäureamid.
aus N-(2-Glycyl-amino-2-desoxy-β-D-glucopyranosyl)-N-octadecyl-ölsäureamid und N-tert.-Butyloxycarbonyl-glycin.
- 17b: N-[2-(N-tert.-Butyloxycarbonyl-L-alanyl-glycyl)-amino-2-desoxy-β-D-glucopyranosyl]-N-octadecyl-ölsäureamid.
aus N-(2-Glycyl-amino-2-desoxy-β-D-glucopyrartosyl)-N-octadecyl-ölsäureamid und N-tert.-Butyloxycarbonyl-L-alanin.
- 17c: N-[2-(N-tert.-Butyloxycarbonyl-O-tert.-butyl-L-aspartyl-glycyl)-amino-2-desoxy-β-D-glucopyranosyl]-N-octadecyl-ölsäureamid.
aus N-(2-Glycyl-amino-2-desoxy-β-D-glucopyranosyl)-N-octadecyl-ölsäureamid und N-tert.-Butyloxycarbonyl-O-tert.-butyl-L-asparaginsäure. Ausbeute 88 %. [α]_{D} = +13,8° (c = 0,93, Dichlormethan).
- 17d: N-[2-(N-tert.-Butyloxycarbonyl-glycyl-glycyl-glycyl)-amino-2-desoxy-β-D-glucopyranosyl]-N-octadecyl-ölsäureamid.
aus N-(2-Glycyl-amino-2-desoxy-β-D-glucopyranosyl)-N-octadecyl-ölsäureamid und N-tert.-Butyloxycarbonyl-glycyl-glycin. Ausbeute 81 %. [α]_{D} = +15,0° (c = 0,94, Dichlormethan).
- 17e: N-[2-(N-tert.-Butyloxycarbonyl-L-alanyl-L-alanyl-glycyl)-amino-2-desoxy-β-D-glucopyranosyl]-N-octadecyl-ölsäureamid.
aus N-(2-Glycyl-amino-2-desoxy-β-D-glucopyranosyl)-N-octadecyl-ölsäureamid und N-tert.-Butyloxycarbonyl-L-alanyl-L-alanin. Ausbeute 55 %. [α]_{D} = +10,3° (c = 0,85, Dichlormethan).
- 17f: N-[2-(N-tert. -Butyloxycarbonyl-glycyl -L-al anyl)-amino-2-desoxy-β-D-glucopyranosyl]-N-octadecyl-ölsäureamid.
aus N-(2-L-Alanyl-amino-2-desoxy-β-D-glucopyranosyl)-N-octadecyl-ölsäureamid und N-tert.-Butyloxycarbonyl-glycin.
- 17g: N-[2-(N-tert.-Butyloxycarbonyl-O-tert.-butyl-L-aspartyl-L-alanyl)-amino-2-desoxy-β-D-glucopyranosyl]-N-octadecyl-ölsäureamid.
aus N-(2-L-Alanyl-amino-2-desoxy-β-D-glucopyranosyl)-N-octadecyl-ölsäureamid und N-tert.-Butyloxycarbonyl-O-tert.-butyl-L-asparaginsäure. Ausbeute 27 %. [α]_{D} = -1,5° (c = 0,55, Dichlormethan).
- 17h: N-[2-(N-tert.-Butyloxycarbonyl-glycyl-glycyl-L-alanyl)-amino-2-desoxy-β-D-glucopyranosyl]-N-octadecyl-ölsäureamid.
aus N-(2-L-Alanyl-amino-2-desoxy-β-D-glucopyranosyl)-N-octadecyl-ölsäureamid und N-tert.-Butyloxycarbonyl-glycyl-glycin. Ausbeute 76 %. [α]_{D} = +14,9° (c = 1,05, Dichlormethan).
- 17i: N-[2-(N-tert.-Butyloxycarbonyl-L-alanyl-L-alanyl-L-alanyl)-amino-2-desoxy-β-D-glucopyranosyl]-N-octadecyl-ölsäureamid.
aus N-(2-L-Alanyl-amino-2-desoxy-β-D-glucopyranosyl)-N-octadecyl-ölsäureamid und N-tert.-Butyloxycarbonyl-L-alanyl-L-alanin. Ausbeute 75 %. [α]_{D} = +17,9° (c = 0,96, Dichlormethan).
- 17j: N-[2-(Di-N-tert.-butyloxycarbonyl-L-lysyl-L-alanyl)-amino-2-desoxy-β-D-glucopyranosyl]-N-octadecyl-ölsäureamid.
aus N-(2-L-Alanyl-amino-2-desoxy-β-D-glucopyranosyl)-N-octadecyl-ölsäureamid und Di-N-tert.-butyloxycarbonyl-L-lysin. Ausbeute 95 %. [α]_{D} = 0,12° (c = 0,84, Dichlormethan).
- 18a: N-[2-N-Carbobenzoxy-glycyl-glycyl)-amino-2-desoxy-β-D-galactopyranosyl]-N-dodecyl-octadecansäureamid.
aus N-(2-Glycyl-amino-2-desoxy-β-D-galactopyranosyl)-N-dodecyl-octadecansäureamid und N-Carbobenzoxy-glycin.
- 18b: N-[2-N-Carbobenzoxy-L-alanyl-glycyl)-amino-2-desoxy-β-D-galactopyranosyl]-N-dodecyl-octadecansäureamid.
aus N-(2-Glycyl-amino-2-desoxy-β-D-galactopyranosyl)-N-dodecyl-octadecansäureamid und N-Carbobenzoxy-L-alanin.

### Beispiel 2

Allgemeine Vorschrift zur Umsetzung der N-geschützten, mit Aminosäuren substituierten 2-Aminoacylamino-2-desoxy-verbindungen der Formel (I) zu den N-unsubstituierten 6-Aminoacylamino-6-desoxy-verbindungen der Formel (I):

Die N-Carbobenzoxy-geschützte Verbindung der allgemeinen Formel (VI) (1,0 mmol) wird in Tetrahydrofuran (10 ml), Methanol (5 ml) und 1N Salzsäure (1 ml) gelöst und mit 10%-iger Palladium-Kohle (0,2 g) versetzt. Die Mischung wird 16 h bei Normaldruck in einer Wasserstoffatmosphäre hydriert. Anschließend wird über eine Celite-Filterschicht abgesaugt und der Rückstand bei vermindertem Druck eingeengt. In vielen Fällen gelingt die Krisallisation aus Metahnol (4 ml) und konz. Ammoniak (0,2 ml). Ansonsten wird der Rückstand wird über Kieselgel säulenchromatographisch gereinigt (Laufmittel Dichlormethan-Methanol-konz. Ammoniak 10:1:0,1). Zur Überführung in das entsprechende Hydrochlorid kann der Rückstand in Tetrahydrofuran (5 ml), Wasser (30 ml) und 1N Salzsäure (1,5 ml) gelöst und gefriergetrocknet werden.

Allgemeine Vorschrift zur Abspaltung der tert.-Butyloxycarbonyl-gruppen in den Verbindungen der Formel (I) zu den Aminen der Formel (I):

Die mit der tert.-Butyloxycarbonyl-gruppe substituierte Verbindung der allgemeinen Formel (VI) (1,0 mmol) wird bei 0° in Dichlormethan (10 ml) gelöst und mit Trifluoressigsäure (10 ml) versetzt. Nach 2 h bei 0° wird mit Toluol (50 ml) verdünnt und bei vermindertem Druck eingeengt. Der erhaltene Rückstand wird dreimal in Toluol (je 50 ml) aufgenommen und jeweils bei vermindertem Druck eingeeng. Der erhaltene Rückstand wird säulenchromatographisch über Kieselgel gereinigt (Gradient Dichlormethan-Methanol-konz. Ammoniak 20:1:0,1 -> 10:1:0,1-> 10:3:0,1). Zur Überführung in das entsprechende Hydrochlorid kann der Rückstand in Tetrahydrofuran (5 ml), Wasser (30 ml) und 1N Salzsäure (1,5 ml) gelöst und gefriergetrocknet werden.
- 21a: N-(2-Glycyl-glycyl-amino-2-desoxy-β-D-glucopyranosyl)-N-dodecyl-dodecansäureamid.
- 21b: N-(2-L-Alanyl-glycyl-amino-2-desoxy-β-D-glucopyranosyl)-N-dodecyldodecansäureamid.
- 21c: N(2-L-Phenylalanyl-glycyl-amino-2-desoxy-β-D-glucopyranosyl)-N-dodecyldodecansäureamid.
- 21d: N-(2-Glycyl-L-alanyl-amino-2-desoxy-β-D-glucopyranosyl)-N-dodecyldodecansäureamid.
- 21e: N-(2-Glycyl-D-alanyl-amino-2-desoxy-β-D-glucopyranosyl)-N-dodecyldodecansäureamid.
- 21f: N-(2-L-Aspartyl-D-alanyl-amino-2-desoxy-β-D-glucopyranosyl)-N-dodecyldodecansäureamid.
- 22a: N-(2-Glycyl-glycyl-amino-2-desoxy-β-D-glucopyranosyl)-N-tetradecyldodecansäureamid.
Ausbeute 91 %. [α]_{D} = +14,9° (c = 0,84, N,N-Dimethylformamid), Schmp. 163° (Zers.).
- 22b: N-(2-Sarcosyl-glycyl-amino-2-desoxy-β-D-glucopyranosyl)-N-tetradecyldodecansäureamid.
Ausbeute 68 %. [α]_{D} = +16,1° (c = 0,93, N,N-Dimethylformamid), Schmp. 116°.
- 22c: N-(2-L-Alanyl-glycyl-amino-2-desoxy-β-D-glucopyranosyl)-N-tetradecyldodecansäureamid.
Ausbeute 90 %. [α]_{D} = +18,7° (c = 0,97, N,N-Dimethylformamid), Schmp. 115-117°.
- 22d: N-(2-L-Valyl-glycyl-amino-2-desoxy-β-D-glucopyranosyl)-N-tetradecyldodecansäureamid.
Ausbeute 90 %. [α]_{D} = +44,7° (c = 0,96, N,N-Dimethylformamid), Schmp. 120° (Zers.).
- 22e: N-(2-L-Seryl-glycyl-amino-2-desoxy-β-D-glucopyranosyl)-N-tetradecyldodecansäureamid.
Ausbeute 68 %. [α]_{D} = +11,3° (c = 0,98, N,N-Dimethylformamid), Schmp. 141-143°.
- 22f: N-(2-L-Glutamyl-glycyl-amino-2-desoxy-β-D-glucopyranosyl)-N-teuadecyldodecansäureamid.
- 22g: N-(2-L-Glutaminyl-glycyl-amino-2-desoxy-β-D-glucopyranosyl)-N-tetradecyldodecansäureamid
- 22h: N-(2-Glycyl-glycyl-glycyl-amino-2-desoxy-β-D-glucopyranosyl)-N-tetradecyldodecansäureamid.
Ausbeute 78 %. [α]_{D} = +17,1° (c = 0,92, N,N-Dimethylformamid), Schmp. 177-178°.
- 22i: N-(2-Glycyl-glycyl-glycyl-glycyl-amino-2-desoxy-β-D-glucopyranosyl)-N-tetradecyl-dodecansäureamid.
- 22j: N-(2-L-Alanyl-glycyl-glycyl-amino-2-desoxy-β-D-glucopyranosyl)-N-tetradecyl-dodecansäureamid.
- 22k: N-(2-L-Alanyl-L-alanyl-glycyl-amino-2-desoxy-β-D-glucopyranosyl)-N-tetradecyl-dodecansäureamid.
Ausbeute 80 %. [α]_{D} = +21,1° (c = 0,90, N,N-Dimethylformamid), Schmp. 132-134°.
- 23a: N-(2-Glycyl-glycyl-amino-2-desoxy-β-D-glucopyranosyl)-N-octadecyldodecansäureamid.
- 23b: N-(2-L-Valyl-glycyl-amino-2-desoxy-β-D-glucopyranosyl)-N-octadecyldodecansäureamid.
- 23c: N-(2-L-Leucyl-glycyl-amino-2-desoxy-β-D-glucopyranosyl)-N-octadecyldodecansäureamid.
- 23d: N-(2-L-Glutamyl-glycyl-amino-2-desoxy-β-D-glucopyranosyl)-N-octadecyldodecansäureamid.
- 23e: N-(2-L-Glutaminyl-glycyl-amino-2-desoxy-β-D-glucopyranosyl)-N-octadecyldodecansäureamid.
- 23f: N-(2-L-Arginyl-glycyl-amino-2-desoxy-β-D-glucopyranosyl)-N-octadecyldodecansäureamid.
- 24a: N-(2-Glycyl-glycyl-amino-2-desoxy-β-D-glucopyranosyl)-N-tetradecyloctadecansäureamid.
- 24b: N-(2-Glycyl-L-alanyl-amino-2-desoxy-β-D-glucopyranosyl)-N-tetradecyloctadecansäureamid.
- 24c: N-(2-L-Leucyl-L-alanyl-amino-2-desoxy-β-D-glucopyranosyl)-N-tetradecyloctadecansäureamid.
- 24d: N-(2-L-Alanyl-L-leucyl-amino-2-desoxy-β-D-glucopyranosyl)-N-tetradecyloctadecansäureamid.
- 24e: N-(2-L-Leucyl-L-leucyl-amino-2-desoxy-β-D-glucopyranosyl)-N-tetradecyloctadecansäureamid.
- 25a: N-(2-Glycyl-glycyl-amino-2-desoxy-β-D-glucopyranosyl)-N-octadecyloctadecansäureamid.
- 25b: N-(2-L-Alanyl-L-alanyl-amino-2-desoxy-β-D-glucopyranosyl)-N-octadecyloctadecansäureamid.
- 25c: N-(2-D-Alanyl-L-alanyl-amino-2-desoxy-β-D-glucopyranosyl)-N-octadecyloctadecansäureamid.
- 25d: N-(2-D-Alanyl-D-alanyl-amino-2-desoxy-β-D-glucopyranosyl)-N-octadecyloctadecansäureamid.
- 25e: N-(2-D-Alanyl-L-alanyl-amino-2-desoxy-β-D-glucopyranosyl)-N-octadecyloctadecansäureamid.
- 25f: N-(2-L-Lysyl-L-alanyl-amino-2-desoxy-β-D-glucopyranosyl)-N-octadecyloctadecansäureamid.
- 26a: N-(2-Glycyl-glycyl-amino-2-desoxy-β-D-glucopyranosyl)-N-tetradecylölsäureamid.
Ausbeute 96 %. [α]_{D} = +13,2° (c = 0,86, Tetrahydrofuran).
- 26b: N-(2-L-Alanyl-glycyl-amino-2-desoxy-β-D-glucopyranosyl)-N-tetradecylölsäureamid.
Ausbeute 97 %. [α]_{D} = +29,6° (c = 0,90, Tetrahydrofuran).
- 26c: N-(2-Glycyl-glycyl-glycyl-amino-2-desoxy-β-D-glucopyranosyl)-N-tetradecylölsäureamid.
Ausbeute 86 %. [α]_{D} = +13,1° (c = 0,94, Tetrahydrofuran).
- 26d: N-(2-L-Alanyl-L-alanyl-glycyl-amino-2-desoxy-β-D-glucopyranosyl)-N-tetradecyl-ölsäureamid.
Ausbeute 97 %. [α]_{D} = +19,9° (c = 0,87, Tetrahydrofuran).
- 26e: N-(2-Glycyl-L-alanyl-amino-2-desoxy-β-D-glucopyranosyl)-N-tetradecylölsäureamid.
Ausbeute 97 %. [α]_{D} = -6,6° (c = 1,05, Tetrahydrofuran).
- 26f: N-(2-L-Alanyl-L-alanyl-amino-2-desoxy-β-D-glucopyranosyl)-N-teuadecylölsäureamid.
Ausbeute 98 %. [α]_{D} = +7,1° (c = 0,87, Tetrahydrofuran).
- 26g: N-(2-L-Leucyl-L-alanyl-amino-2-desoxy-β-D-glucopyranosyl)-N-tetradecylölsäureamid.
Ausbeute 96 %. [α]_{D} = -1,6° (c = 0,88, Tetrahydrofuran).
- 26h: N-(2-Glycyl-glycyl-L-alanyl-amino-2-desoxy-β-D-glucopyranosyl)-Ntetradecyl-ölsäureamid.
Ausbeute 96 %. [α]_{D} = +8,1° (c = 0,97, Tetrahydrofuran).
- 26i: N-(2-Glycyl-L-leucyl-amino-2-desoxy-β-D-glucopyranosyl)-N-tetradecylölsäureamid.
Ausbeute 97 %. [α]_{D} = -9,1° (c = 1,02, Tetrahydrofuran).
- 26j: N-(2-L-Alanyl-L-leucyl-amino-2-desoxy-β-D-glucopyranosyl)-N-tetradecylölsäureamid.
Ausbeute 87 %. [α]_{D} = +1,0° (c = 0,74, Tetrahydrofuran).
- 26k: N-(2-Glycyl-glycyl-L-leucyl-amino-2-desoxy-β-D-glucopyranosyl)-N-tetradecyl-ölsäureamid.
Ausbeute 95 %. [α]_{D} = -2,90 (c = 0,87, Tetrahydrofuran).
- 27a: N-(2-Glycyl-glycyl-amino-2-desoxy-β-D-glucopyranosyl)-N-octadecylölsäureamid.
- 27b: N-(2-L-Alanyl-glycyl-amino-2-desoxy-β-D-glucopyranosyl)-N-octadecylölsäureamid.
- 27c: N-(2-L-Aspartyl-glycyl-amino-2-desoxy-β-D-glucopyranosyl)-N-octadecylölsäureamid.
Ausbeute 92 %. [α]_{D} = +11,3° (c = 0,90, N,N-Dimethylformamid).
- 27d: N-(2-Glycyl-glycyl-glycyl-amino-2-desoxy-β-D-glucopyranosyl)-N-octadecylölsäureamid.
Ausbeute 94 %. [α]_{D} = +12,5° (c = 0,93, Methanol).
- 27e: N-(2-L-Alanyl-L-alanyl-glycyl-amino-2-desoxy-β-D-glucopyranosyl)-N-octadecyl-ölsäureamid.
Ausbeute 91 %. [α]_{D} = +14,3° (c = 0,92, Methanol).
- 27f: N-(2-Glycyl-L-alanyl-amino-2-desoxy-β-D-glucopyranosyl)-N-octadecylölsäureamid.
- 27g: N-(2-L-Aspartyl-L-alanyl-amino-2-desoxy-β-D-glucopyranosyl)-N-octadecylölsäureamid.
Ausbeute 88 %. [α]_{D} = +35,6° (c = 0,76, Dichlormethan).
- 27h: N-(2-Glycyl-glycyl-L-alanyl-amino-2-desoxy-β-D-glucopyranosyl)-N-octadecyl-ölsäureamid.
Ausbeute 96 %. [α]_{D} = -57,1° (c = 0,77, Dichlormethan).
- 27i: N-(2-L-Alanyl-L-alanyl-L-alanyl-amino-2-desoxy-β-D-glucopyranosyl)-N-octadecyl-ölsäureamid.
Ausbeute 85 %. [α]_{D} = +7,4° (c = 0,82, Dichlormethan).
- 27j: N-(2-L-Lysyl-L-alanyl-amino-2-desoxy-β-D-glucopyranosyl)-N-octadecylölsäureamid.
Ausbeute 84 %. [α]_{D} = +4,6° (c = 1,09, Methanol).
- 28a: N-(2-Glycyl-glycyl-amino-2-desoxy-β-D-galactopyranosyl)-N-dodecyloctadecansäureamid.
- 28b: N-(2-L-Alanyl-glycyl-amino-2-desoxy-β-D-galactopyranosyl)-N-dodecyloctadecansäureamid.

## Patentansprüche

1. Aminosäure substituierte (2-Aminoacylamino-2-desoxy-glycosyl)-amide der allgemeinen Formel (I), 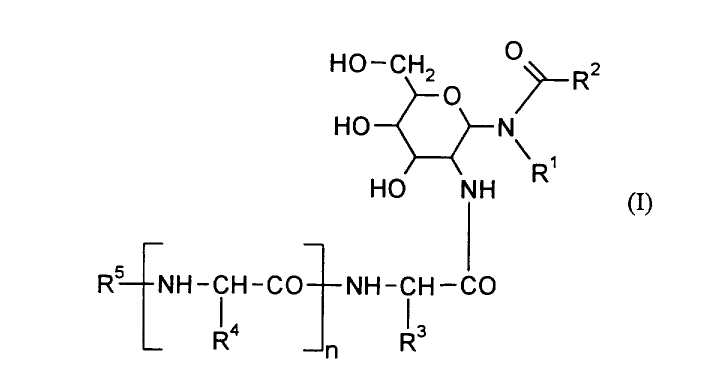 in welcher
R¹ für geradkettiges oder verzweigtes, gesättigtes oder ungesättigtes Alkyl mit bis zu 25 Kohlenstoffatomen steht,
R² für geradkettiges oder verzweigtes, gesättigtes oder ungesättigtes Alkyl mit bis zu 25 Kohlenstoffatomen steht,
R³ für Wasserstoff, C₁- bis C₇-Alkyl, Hydroxy-methyl, 1-Hydroxy-ethyl, Mercapto-methyl, 2-Methylthio-ethyl, 3-Amino-propyl, 3-Ureidopropyl, 3-Guanidyl-propyl, 4-Amino-butyl, Carboyxy-methyl, Carbamoyl-methyl, 2-Carboxy-ethyl, 2-Carbamoyl-ethyl, Benzyl, 4-Hydroxy-benzyl, 3-Indolyl-methyl oder 4-Imidazolyl-methyl steht,
R⁴ die oben angegebene Bedeutung von R³ hat und mit dieser gleich oder verschiedenen ist,
R⁵ für Wasserstoff oder eine in der Peptidchemie übliche Schutzgruppe steht,
und worin
n eine Zahl 1, 2 oder 3 bedeutet,
wobei für den Fall, daß n = 2 oder 3 ist, die einzelnen Bedeutungen von R⁴ verschieden sein können.

2. Verbindungen der allgemeinen Formel (I) gemäß Anspruch 1, in welcher
R¹ für einen geradkettigen, gesättigten oder einfach ungesättigten Alkylrest mit 10 bis 20 Kohlenstoffatomen steht,
R² für einen geradkettigen, gesättigten oder einfach ungesättigten Alkylrest mit 10 bis 20 Kohlenstoffatomen steht,
R³ für Wasserstoff, C₁- bis C₇-Alkyl, Hydroxy-methyl, 1-Hydroxy-ethyl, Mercapto-methyl, 2-Methylthio-ethyl, 3-Amino-propyl, 3-Ureidopropyl, 3-Guanidyl-propyl, 4-Amino-butyl, Carboyxy-methyl, Carbamoyl-methyl, 2-Carboxy-ethyl, 2-Carbamoyl-ethyl, Benzyl, 4-Hydroxy-benzyl, 3-Indolyl-methyl oder 4-Imidazolyl-methyl steht,
R⁴ die oben angegebene Bedeutung von R³ hat und mit dieser gleich oder verschiedenen ist,
R⁵ für Wasserstoff, Acetyl, Benzoyl, Trichloracetyl, Trifluoracetyl, Methoxycarbonyl, tert.-Butyloxycarbonyl, Allyloxycarbonyl, Trichlorethoxycarbonyl, Benzyloxycarbonyl oder Fluorenylmethyoxycarbonyl steht,
und worin
n eine Zahl 1, 2 oder 3 bedeutet,
wobei für den Fall, daß n = 2 oder 3 ist, die einzelnen Bedeutungen von R⁴ verschieden sein können.

3. Verfahren zur Herstellung der Verbindungen der allgemeinen Formel (I) gemäß Anspruch 1, dadurch gekennzeichnet, daß man
a) N-(2-Aminoacylamino-2-desoxy-hexopyranosyl)-N-alkyl-carbonsäureamide der Formel (II) 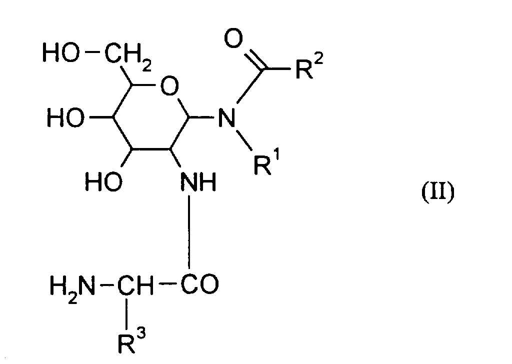 in welcher
R¹, R² und R³ die in den Ansprüchen 1 und 2 angegebene Bedeutung haben,
mit Aminosäure-, Di- oder Tripeptid-derivaten der allgemeinen Formel (III) 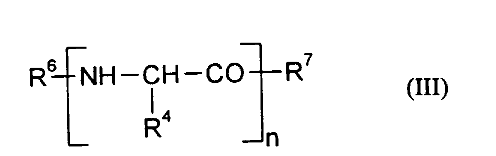 in welcher
R⁴ die in den Ansprüchen 1 und 2 angegebene Bedeutung hat,
R⁶ eine in der Peptidchemie übliche Schutzgruppe für das Stickstoff-atom von Aminosäuren darstellt, die selektiv unter Erhalt der Peptidbindung wieder abgespalten werden kann, und
R⁷ eine Hydroxygruppe oder eine in der Peptidchemie übliche Fluchtgruppe für die Aktivierung von Aminosäuren darstellt,
n eine Zahl 1, 2 oder 3 bedeutet,
wobei für den Fall, daß n = 2 oder 3 ist, die einzelnen Bedeutungen von R⁴ verschieden sein können,
miteinander derart zur Reaktion bringt, daß eine amidische Bindung geknüpft wird und Verbindungen der allgemeinen Formel (I) erhalten werden und
b) anschließend die Aminoschutzgruppen R⁶ abgespalten werden, wobei Verbindungen mit unsubstituierter Aminogruppe der allgemeinen Formel (I) erhalten werden.

4. Verfahren zur Herstellung von Verbindungen der allgemeinen Formel (I) gemäß Anspruch 1, dadurch gekennzeichnet, daß man
a) Verbindungen der allgemeinen Formel (IV) 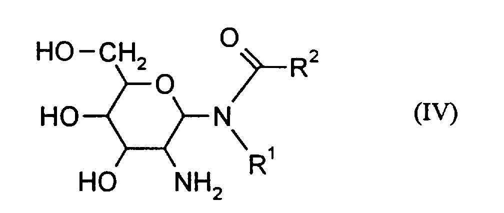 in welcher
R¹ und R² die in den Ansprüchen 1 und 2 angegebene Bedeutung haben
mit Derivaten von Di-, Tri- oder Tetrapeptiden der allgemeinen Formel (III) 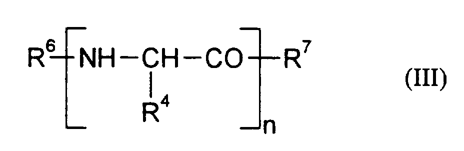 in welcher
R⁴ und R⁵ die in den Ansprüchen 1 und 2 angegebene Bedeutung haben und
R⁶ eine Schutzgruppe bedeutet
und worin
n eine Zahl 2, 3 oder 4 bedeutet,
wobei für den Fall, daß n = 2 oder 3 ist, die einzelnen Bedeutungen von R⁴ verschieden sein können,
miteinander unter den oben genannten Bedingungen unter Ausbildung einer peptidischen Bindung zu Reaktion bringt, wobei die an der N-terminalen Aminogruppe substituierten Verbindungen der Formel (V) 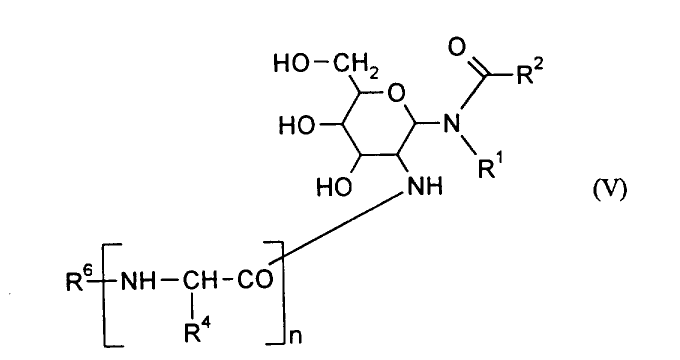 worin R¹, R², R⁴ und R⁶ die in den Ansprüchen 1 und 2 angegebenen Bedeutungen haben, und
n eine Zahl 2, 3 oder 4 bedeutet,
erhalten werden und
b) anschließend die Amino-schutzgruppen R⁶ abzuspalten, wobei Verbindungen mit unsubstituierter Aminogruppe der allgemeinen Formel (I) erhalten werden.

5. Arzneimittel enthaltend eine oder mehrere Verbindungen aus den Ansprüchen 1 und 2.

6. Verwendung von Verbindungen aus den Ansprüchen 1 und 2 zur Herstellung von Arzneimitteln.

## Claims

1. (2-Aminoacylamino-2-deoxy-glycosyl)-amides substituted by amino acid, of the general formula (I) 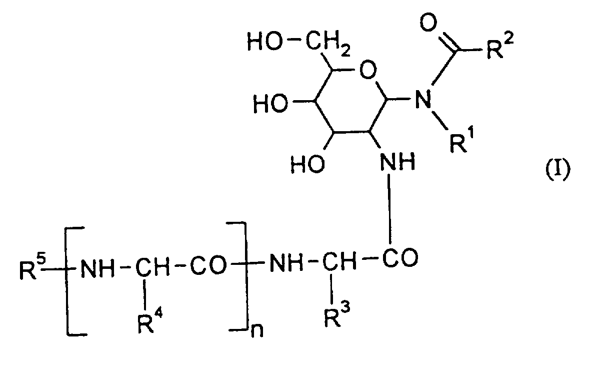 in which
R¹ represents straight-chain or branched, saturated or unsaturated alkyl having up to 25 carbon atoms,
R² represents straight-chain or branched, saturated or unsaturated alkyl having up to 25 carbon atoms,
R³ represents hydrogen, C₁- to C₇-alkyl, hydroxy-methyl, 1-hydroxy-ethyl, mercapto-methyl, 2-methylthio-ethyl, 3-amino-propyl, 3-ureido-propyl, 3-guanidyl-propyl, 4-amino-butyl, carboxy-methyl, carbamoyl-methyl, 2-carboxy-ethyl, 2-carbamoyl-ethyl, benzyl, 4-hydroxy-benzyl, 3-indolylmethyl or 4-imidazolyl-methyl,
R⁴ has the above-indicated meaning of R³ and is identical to or different from it,
R⁵ represents hydrogen or a protecting group which is customary in peptide chemistry,
and in which
n denotes a number 1, 2 or 3,
and in the case where n = 2 or 3 the individual meanings of R⁴ can be different.

2. Compounds of the general formula (I) according to Claim 1, in which
R¹ represents a straight-chain, saturated or monounsaturated alkyl radical having 10 to 20 carbon atoms,
R² represents a straight-chain, saturated or monounsaturated alkyl radical having 10 to 20 carbon atoms,
R³ represents hydrogen, C₁- to C₇-alkyl, hydroxy-methyl, 1-hydroxy-ethyl, mercapto-methyl, 2-methylthio-ethyl, 3-amino-propyl, 3-ureido-propyl, 3-guanidyl-propyl, 4-amino-butyl, carboxy-methyl, carbamoyl-methyl, 2-carboxy-ethyl, 2-carbamoyl-ethyl, benzyl, 4-hydroxy-benzyl, 3-indolylmethyl or 4-imidazolyl-methyl,
R⁴ has the above-indicated meaning of R³ and is identical to or different from it,
R⁵ represents hydrogen, acetyl, benzoyl, trichloroacetyl, trifluoroacetyl, methoxycarbonyl, tert-butyloxycarbonyl, allyloxycarbonyl, trichloroethoxycarbonyl, benzyloxycarbonyl or fluorenylmethoxycarbonyl,
and in which
n denotes a number 1, 2 or 3,
and in the case where n = 2 or 3 the individual meanings of R⁴ can be different.

3. Process for the preparation of the compounds of the general formula (I) according to Claim 1, characterized in that
a) N-(2-aminoacylamino-2-deoxy-hexopyranosyl)-N-alkyl-carboxamidesof the formula (II) 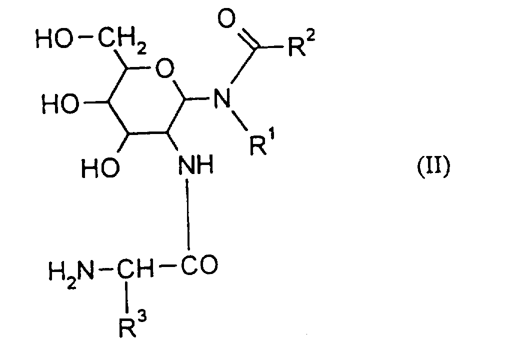 in which
R¹, R² and R³ have the meaning given in Claims 1 and 2
are reacted with amino acid derivatives or di- or tripeptide derivatives of the general formula (III) 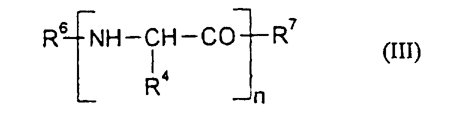 in which
R⁴ has the meaning given in Claims 1 and 2,
R⁶ represents a protecting group, customary in peptide chemistry, for the nitrogen atom of amino acids, which can be eliminated again selectively to give the peptide linkage, and
R⁷ represents a hydroxyl group or a leaving group, customary in peptide chemistry, for the activation of amino acids,
n denotes a number 1, 2 or 3,
and in the case where n is 2 or 3 the individual meanings of R⁴ can be different,
with one another in such a way that an amide linkage is formed and compounds of the general formula (I) are obtained and
b) subsequently the amino-protecting groups R⁶ are eliminated to give compounds having an unsubstituted amino group, of the general formula (I).

4. Process for the preparation of compounds of the general formula (I) according to Claim 1, characterized in that
a) compounds of the general formula (IV) 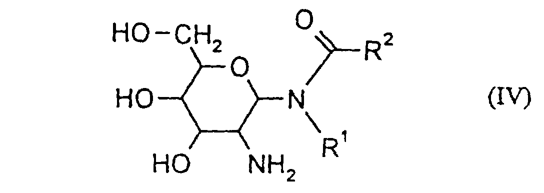 in which
R¹ and R² have the meaning given in Claims 1 and 2
are reacted with derivatives of di-, tri- or tetrapeptides of the general formula (III) 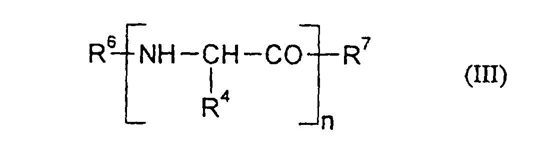 in which
R⁴ and R⁷ have the meaning in Claims 1 to 3 and
R⁶ denotes a protecting group
and in which
n denotes a number 2, 3 or 4
and in the case where n is 2 or 3 the individual meanings of R⁴ can be different
with one another under the abovementioned conditions with formation of a peptide linkage, to give the compounds of the formula (V) 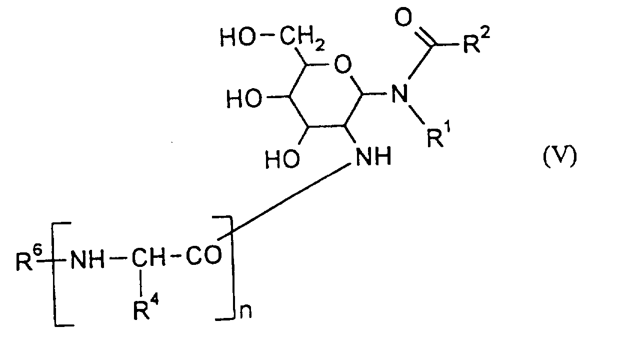 in which R¹, R², R⁴ and R⁶ have the meanings given in Claims 1 to 3 and
n denotes a number 2, 3 or 4,
which are substituted on the N-terminal amino group,
and
b) subsequently the amino-protecting groups R⁶ are eliminated to give compounds having an unsubstituted amino group, of the general formula (I).

5. Medicaments comprising one or more compounds of Claims 1 and 2.

6. Use of compounds of Claims 1 and 2 for the preparation of medicaments.

## Revendications

1. (2-aminoacylamino-2-désoxy-glycosyl)-amides substitués sur l'amino-acide, de formule générale (I), 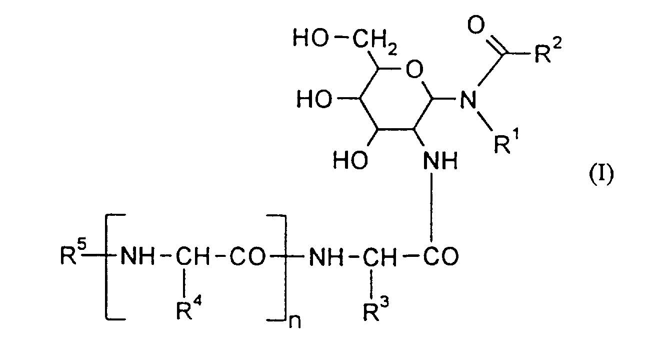 dans laquelle
R¹ est un groupe alkyle saturé ou non saturé linéaire ou ramifié ayant jusqu'à 25 atomes de carbone,
R² est un groupe alkyle saturé ou non saturé linéaire ou ramifié ayant jusqu'à 25 atomes de carbone,
R³ représente de l'hydrogène, un groupe alkyle en C₁ à C₇, hydroxyméthyle, 1-hydroxyéthyle, mercaptométhyle, 2-méthylthio-éthyle, 4-aminopropyle, 3-uréidopropyle, 3-guanidylpropyle, 4-aminobutyle, carboxyméthyle, carbamoylméthyle, 2-carboxyéthyle, 2-carbamoyléthyle, benzyle, 4-hydroxybenzyle, 3-indolylméthyle ou 4-imidazolylméthyle,
R⁴ a la définition donnée ci-dessus pour R³ et est identique à R³ ou en est différent,
R⁵ représente de l'hydrogène ou un groupe protecteur classique dans la chimie des peptides,
et dans laquelle
n a la valeur 1, 2 ou 3,
les définitions individuelles de R⁴ pouvant être différentes au cas où n a la valeur 2 ou 3.

2. Composés de formule générale (I) suivant la revendication 1, dans laquelle
R¹ est un reste alkyle linéaire, saturé ou mono-insaturé ayant 10 à 20 atomes de carbone,
R² représente un reste alkyle linéaire, saturé ou mono-insaturé ayant 10 à 20 atomes de carbone,
R³ représente de l'hydrogène, un groupe alkyle en C₁ à C₇, hydroxyméthyle, 1-hydroxyéthyle, mercaptométhyle, 2-méthylthio-éthyle, 3-aminopropyle, 3-uréidopropyle, 3-guanidylpropyle, 4-aminobutyle, carboxyméthyle, carbamoylméthyle, 2-carboxyéthyle, 2-carbamoyléthyle, benzyle, 4-hydroxybenzyle, 3-indolylméthyle ou 4-imidazolylméthyle,
R⁴ a la définition indiquée ci-dessus pour R³ et est identique à R³ ou en est différent,
R⁵ représente de l'hydrogène, un groupe acétyle, benzoyle, trichloracétyle, trifluoracétyle, méthoxycarbonyle, tertio-butyloxycarbonyle, allyloxycarbonyle, trichloréthoxycarbonyle, benzyloxycarbonyle ou fluorénylméthoxycarbonyle,
et dans laquelle
n a la valeur 1, 2 ou 3,
les valeurs individuelles de R⁴ pouvant être différentes au cas où n a la valeur 2 ou 3.

3. Procédé de production des composés de formule générale (I) suivant la revendication , caractérisé en ce que
a) on fait réagir ensemble des N-(2-aminoacylamino-2-désoxy-hexopyrannosyl)-N-alkyl-carboxamides de formule (II) 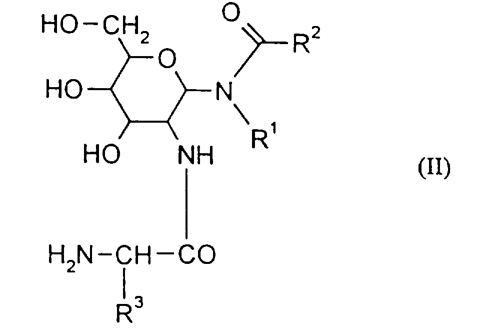 dans laquelle
R¹, R² et R³ ont la définition indiquée dans les revendications 1 et 2,
avec des dérivés d'amino-acides, de dipeptides ou de tripeptides de formule générale (III) 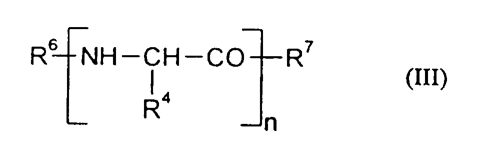 dans laquelle
R⁴ a la définition indiquée dans les revendications 1 et 2,
R⁶ représente un groupe protecteur, classique dans la chimie des peptides, pour l'atome d'azote d'amino-acides, qui peut être éliminé sélectivement avec maintien de la liaison peptidique et
R⁷ représente un groupe hydroxy ou un groupe partant, d'emploi classique dans la chimie des peptides, pour l'activation d'amino-acides,
n est le nombre 1, 2 ou 3,
les définitions individuelles de R⁴ pouvant être différentes au cas où n a la valeur 2 ou 3,
de façon telle que la liaison amidique soit 5 créée et que l'on obtienne des composés de formule générale (I), puis
b) on élimine les groupes R⁶ protégeant la fonction amino, et on obtient ainsi des composés de formule générale (I) à groupe amino non substitué.

4. Procédé de production de composés de formule générale (I) suivant la revendication 1, caractérisé en ce que
a) on fait réagir ensemble des composés de formule générale (IV) 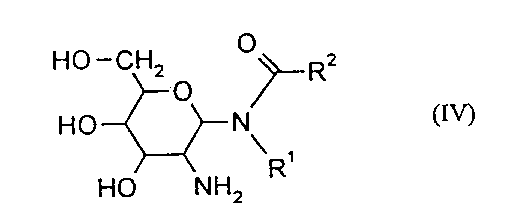 dans laquelle
R¹ et R² ont la définition indiquée dans les revendications 1 et 2
avec des dérivés de dipeptides, tripeptides ou tétrapeptides de formule générale (III) 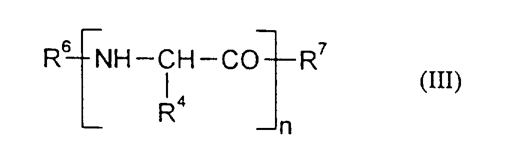
dans laquelle
R⁴ et R⁵ ont la définition indiquée dans les revendications 1 et 2 et
R⁶ est un groupe protecteur et dans laquelle
n représente le nombre 2, 3 ou 4,
les définitions individuelles de R⁴ pouvant être différentes au cas où n a la valeur 2 ou 3,
dans les conditions mentionnées ci-dessus avec formation d'une liaison peptidique, et on obtient alors les composés, substitués sur le groupe amino N-terminal, de formule (V) 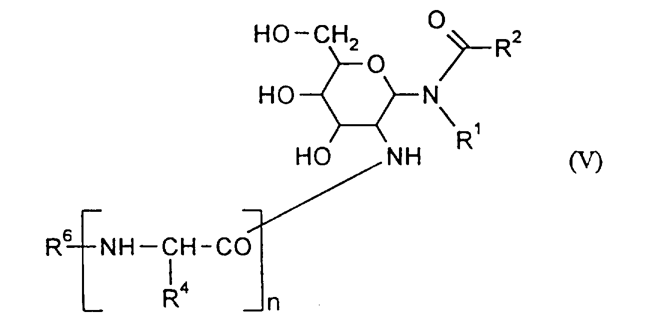 dans laquelle R¹, R², R⁴ et R⁶ ont les définitions dans les revendications 1 et 2, et n est le nombre 2, 3 ou 4,
puis
b) on élimine les groupes R⁶ protégeant la fonction amino et on obtient ainsi des composés de formule générale (I) à groupe amino non substitué.

5. Médicament contenant un ou plusieurs composés suivant les revendications 1 et 2.

6. Utilisation de composés suivant les revendications 1 et 2 pour la préparation de médicaments.
